# EUROPEAN PATENT APPLICATION

(11) **EP 3 978 502 A1**
(43) Date of publication of application: **06.04.2022**
(21) Application number: 20814492.3
(22) Date of filing: 25.03.2020
(51) Int. Cl.: C07D 495/04, A61K 31/519, A61P 37/00

(54) **COMPOUND FOR INHIBITING ROR?T ACTIVITY, PREPARATION METHOD THEREFOR AND APPLICATION THEREOF**

(30) Priority: 29.05.2019 CN 201910456766
(71) Applicant: Cellregen (Beijing) Life Science and Technology Co., Ltd., Beijing 102200 (CN)
(72) Inventor: HUANG, Zhaofeng, Guangzhou, Guangdong 510080 (CN); BAI, Chuan, Guangzhou, Guangdong 510080 (CN)
(74) Representative: Stafford, Jonathan Alan Lewis
(86) International application number: PCT/CN2020/081051
(87) International publication number: WO 2020/238369

(57) **Abstract**

The present invention relates to a compound for inhibiting RORyt activity, a preparation method therefor and an application thereof. The compound for inhibiting RORyt activity has the following structural formula (A): 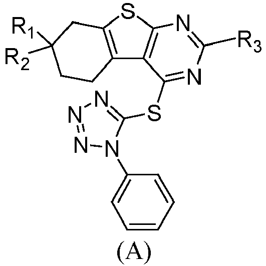 it has been discovered by means of researching the present invention that the compound that has the structural formula (A) has a good inhibitory effect on RORyt activity. Specifically, luciferase activity reporter system and Th17 cell differentiation experiments prove that the compound may inhibit the transcription of a key transcription factor RORyt for Th7 cell differentiation, and may inhibit the transcriptional expression of target gene IL17A and IL17F molecules of RORyt. The intracellular expression of IL17A at the protein level as detected by a flow cytometer is also significantly inhibited, proving that the compound molecule has an obvious inhibitory effect on Th17 cell activity. Therefore, the compound may be used to produce corresponding activity inhibitors, and provide a leading structure for the development of therapeutic drugs for autoimmune diseases.

## Description

### TECHNICAL FIELD

The present disclosure relates to the technical field of pharmaceutical synthesis, in particular to a compound for inhibiting RORyt activity, a preparation method therefor and application thereof.

### BACKGROUND

At present, autoimmune diseases affect 5% of the world's population. More than 70 kinds of human diseases, including psoriasis, multiple sclerosis, rheumatoid arthritis, asthma, and inflammatory bowel disease, are associated with autoimmune disorders (Goodnow et al., 2005). At present, the treatment of autoimmune diseases mainly relies on some non-selective immunosuppressants, which have limited efficacy and large side effects. Therefore, there are no very specific drugs for the treatment of autoimmune diseases in clinical practice. Therefore, the development of new autoimmune drugs with high efficacy and low side effects has become an urgent clinical need.

Studies have shown that a T cell subset, Th17, is associated with the occurrence of human autoimmune diseases and related animal models (Linden 2006; Kikly et al., 2006). In rheumatoid arthritis, psoriasis, asthma, inflammatory bowel disease and other autoimmune diseases, the expression of IL17, a characteristic cytokine of the Th17 cells, is up-regulated, and inhibiting the expression of IL17 and the formation of Th17 cells can reduce the occurrence or clinical severity of autoimmune diseases (Bowman et al., 2006; Kikly et al., 2006). Existing studies have shown that the differentiation and formation of the Th17 cell are controlled by a transcription factor RORyt. In RORyt knockout mice, the differentiation ability of Th17 cells is reduced, and the number of Th17 cells is reduced, and the probability of induced experimental allergic encephalomyelitis (EAE) autoimmune disease in mice and clinical scoring index is significantly reduced (Ivanov et al., 2006). This means that a RORyt functional inhibitor can be used as a directional target for the development of therapeutic drugs for Th17-mediated autoimmune diseases.

RORyt is a member of steroid nuclear receptor family, and its protein molecule includes a conserved DNA binding domain and a ligand binding domain composed of 12 helices. The ligand binding domain is an important region for ligand binding, nuclear localization, and dimer forming. Steroid receptor co-activator molecules (SRCs) can bind to AF2 region of a ligand domain to depolymerize a transcription inhibitory complex, recruit transcription activator molecules, and initiate the transcription of related genes (Glass and Rosenfeld, 2000). At present, a natural ligand of RORyt has not been found, but two recent studies have found that a synthetic molecule digoxin and derivatives thereof (Huh et al., 20011; Fujita-Sato et al., 2011) and SR1001 (Solt et al., 2011) can specifically bind to the ligand domain of RORyt to inhibit the function of RORyt and reduce the differentiation ability of Th17 cells, and alleviation the clinical symptoms of autoimmune disease EAE in mice. However, digoxin and derivatives thereof are very toxic (Paula et al., 2005), and SR1001 molecule, even administrated at high concentrations (40 mg /kg), can only reduce the clinical symptoms of EAE slightly in vivo although it can effectively inhibit the differentiation of Th17 cells in vitro. In addition, SR1001 can interact with other RORs, thus has poor selectivity (Solt et al., 2011). On the one hand, these studies show that inhibiting the function of RORyt to treat autoimmune diseases is a feasible solution, but an ideal target drug has not yet been found. Therefore, looking for an RORyt functional inhibitor with high efficiency, low toxicity, and strong specificity will be an important aspect for the development of drugs for Th17-mediated autoimmune diseases.

### SUMMARY

Therefore, it is necessary to provide a compound for inhibiting RORyt activity, a preparation method therefor and application thereof.

A compound for inhibiting RORyt activity has the following structural formula (A): 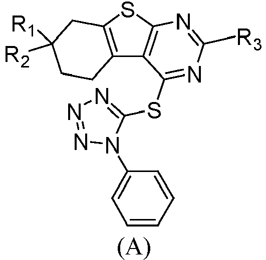 where R₁ and R₂ are one each independently selected from the group consisting of hydrogen, fluorine, chlorine, bromine, iodine, a C1 to C4 linear or branched alkyl group, 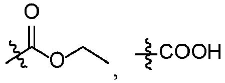 and 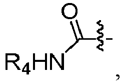 R₄ is selected from the group consisting of 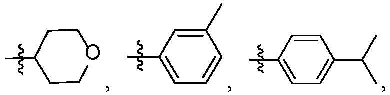 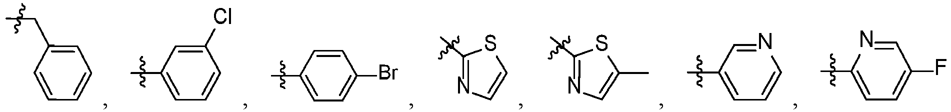 and 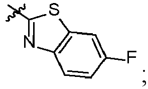 and R₃ is selected from the group consisting of methyl,

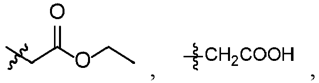

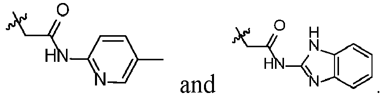

In one embodiment, R₃ is methyl, R₁ and R₂ are not simultaneously hydrogen, and when one of R₁ and R₂ is hydrogen, the other is not methyl.

In one embodiment, one of R₁ and R₂ is hydrogen, and the other is selected from the group consisting of a C2 to C4 linear or branched alkyl group, 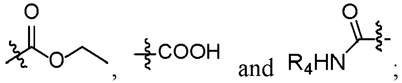 or R₁ and R₂ are both methyl; or
R₁ and R₂ are independently selected from the group consisting of fluorine, chlorine, bromine and iodine.

In one embodiment, R₁ and R₂ are simultaneously hydrogen, and R₃ is selected from the group consisting of

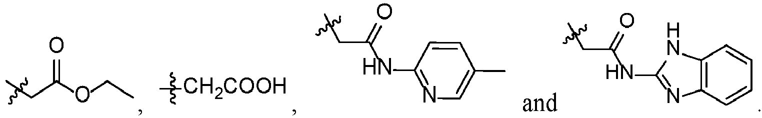

In one embodiment, the compound represented by the structural formula (A) is one selected from the following compounds:

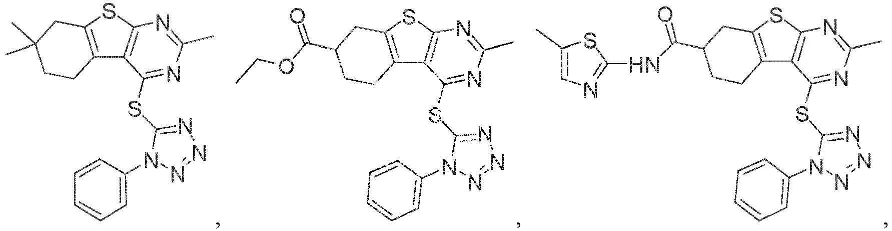

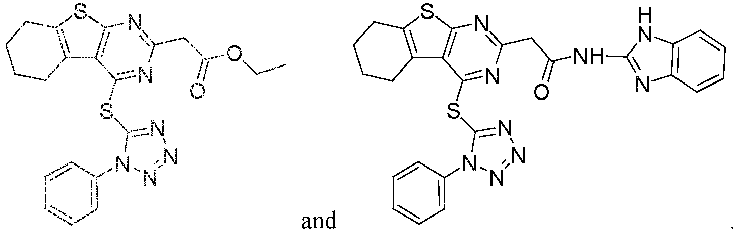

A method for preparing a compound for inhibiting RORyt activity includes the following steps: reacting compounds 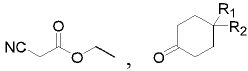 with elemental sulfur in the presence of morpholine and ethanol to prepare a compound 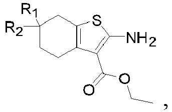 where R₁ and R₂ are each independently selected from the group consisting of hydrogen, fluorine, chlorine, bromine, iodine, a C1 to C4 linear or branched alkyl group, 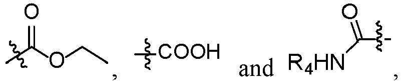 R₄ is selected from the group consisting of 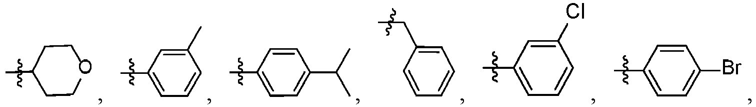 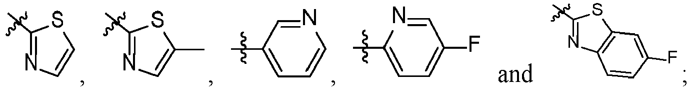 reacting the prepared compound 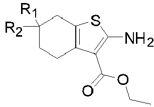 with NC-R₃ in a solution of hydrogen chloride in 1,4-dioxane to prepare a compound 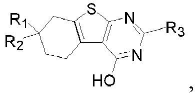 where R₃ is selected from the group consisting of methyl, 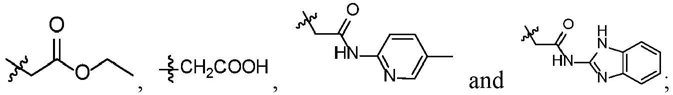 reacting the prepared compound 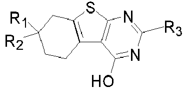 with phosphorus oxychloride to prepare a compound 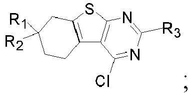 and reacting the prepared compound 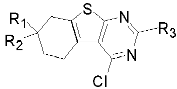 with a compound 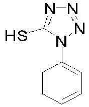 in the presence of sodium bicarbonate and dimethyl sulfoxide to prepare a compound

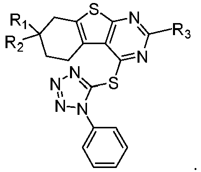

In one embodiment, the compound sodium azide in an aqueous solution. 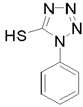 is prepared by reacting phenyl thioisocyanate and

In one embodiment, one of R₁ and R₂ is hydrogen, and the other is 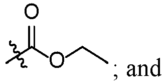 the preparation method further includes reacting a compound 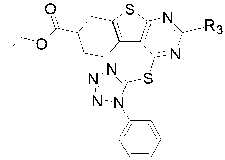 in a lithium hydroxide aqueous solution to prepare a compound

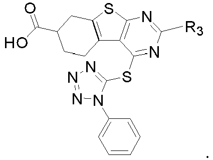

In one embodiment, one of R₁ and R₂ is hydrogen, and the other is 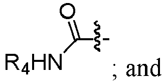 the preparation method further includes reacting the prepared compound 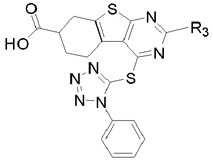 with R₄NH₂, HOBT and EDCI in an ultra-dry DCM solvent to prepare a compound

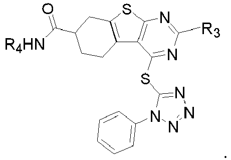

Use of the compound for inhibiting RORyt activity as described in any of the above embodiments in preparation of a therapeutic formulation for treatment of autoimmune diseases, in preparation of a formulation that inhibits formation of a Th17 cell, in preparation of a formulation that inhibits RORyt transcription activity, or in preparation of a formulation that inhibits transcription and expression of IL17A gene and/or IL17F gene.

In the present disclosure, it has been found by means of researching that the compounds having the structural formula (A) have a good inhibitory effect on RORyt activity. Specifically, luciferase activity reporter system and Th17 cell differentiation experiments prove that the compound may inhibit the transcription effect of a key transcription factor RORyt for Th7 cell differentiation, and may inhibit the transcription and expression of genes IL17A and IL17F targeted by RORyt. The intracellular expression of IL17A at the protein level is also significantly inhibited as detected by the flow cytometer, indicating that the compound molecule has an obvious inhibitory effect on Th17 cell activity. Therefore, the compound may be used to produce a formulation of corresponding activity inhibitor, and provide a leading structure for the development of therapeutic drugs for autoimmune diseases.

In particular, in the present disclosure, seed compounds of the compound represented by structural formula (A) have been further researched and subjected to substitution. For some of the substituents, creative designs and studies have been conducted. Some compounds that have significant inhibitory effects on RORyt activity have been found from numerous compounds. The research on these compounds can further provide more reliable technical support for leading drugs and even candidate drugs of the therapeutic drugs for autoimmune diseases.

### BRIEF DESCRIPTION OF THE DRAWINGS

FIG. 1, FIG. 2, and FIG. 3 show schematic diagrams of synthetic routes of different intermediates and compounds, respectively.
FIG. 4, FIG. 6, FIG. 8, FIG. 10, FIG. 12, and FIG. 17 show ¹H NMR spectra of Compounds 6, 10, 13, 22, 28, and 31, respectively.
FIG. 5, FIG. 7, FIG. 9, FIG. 11, FIG. 13, and FIG. 18 show ¹³C NMR spectra of Compounds 6, 10, 13, 22, 28, and 31, respectively.
FIG. 14, FIG. 15, FIG. 16, and FIG. 19 show HR-ESI-MS spectra of Compounds 28, 29, 30, and 31, respectively.
FIG. 20 shows results of RORyt transcription activity inhibited by Compounds 28 and 31.
FIG. 21 shows data of intracellular protein expression of IL-17A inhibited by Compounds 6, 10, 22, 28, and 31.
FIG. 22 shows results of mRNA expression of RORyt (left), IL17A (middle), and IL-17F (right) inhibited by Compounds 6, 10, 22, 28, and 31 in the differentiation induced by Th17 cells in vitro.

### DETAILED DESCRIPTION OF THE EMBODIMENTS

For the convenience of understanding the present disclosure, embodiments of the disclosure are described more fully hereinafter with reference to the accompanying drawings. Preferable embodiments of the present disclosure are presented in the drawings. However, the present disclosure may be embodied in many different forms and is not limited to the embodiments described herein. Rather, these embodiments are provided so that the understanding of the disclosure of the present disclosure will be more thorough and complete.

Unless otherwise defined, all technical and scientific terms used herein have the same meaning as commonly understood by skilled person in the art to which this disclosure belongs. The terms used in the specification of the present disclosure herein are for the purpose of describing specific embodiments only and are not intended to limit the present disclosure. The term "and/or" used herein includes any and all combinations of one or more of the associated listed items.

The present disclosure provides a compound for inhibiting RORyt activity having the following structural formula (A): 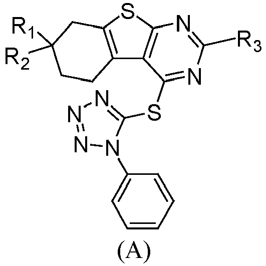 where R₁ and R₂ are one each independently selected from the group consisting of hydrogen, fluorine, chlorine, bromine, iodine, a C1 to C4 linear or branched alkyl group, 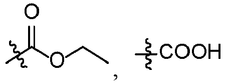 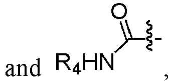 R₄ is selected from the group consisting of 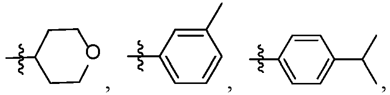 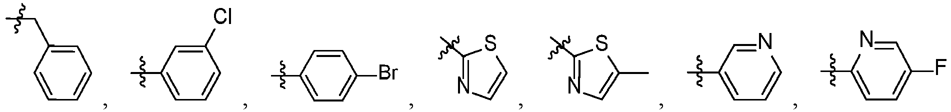 and 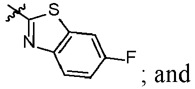 R₃ is selected from the group consisting of methyl,

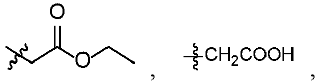

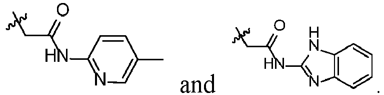

In one specific embodiment, R₃ is methyl, R₁ and R₂ are not simultaneously hydrogen, and when one of R₁ and R₂ is hydrogen, the other is not methyl.

More specifically, one of R₁ and R₂ is hydrogen, and the other is selected from the group consisting of a C2 to C4 linear or branched alkyl group, 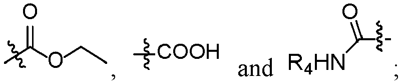 or R₁ and R₂ are independently selected from the group consisting of fluorine, chlorine, bromine and iodine.

In another specific embodiment, R₁ and R₂ are simultaneously hydrogen, and R₃ is selected from the group consisting of

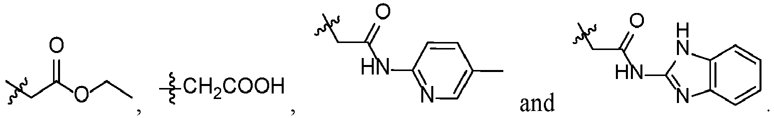

In one specific embodiment, the compound for inhibiting RORyt activity may be one selected from the group consisting of:

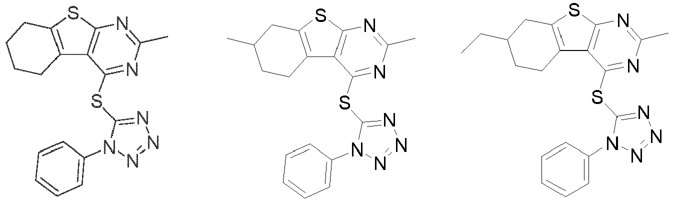

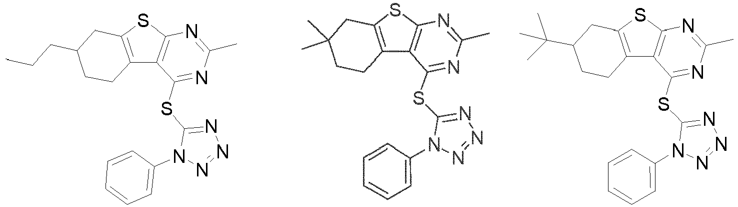

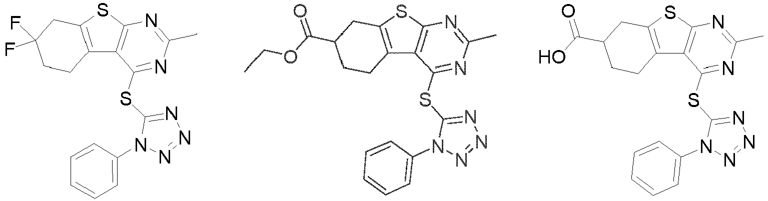

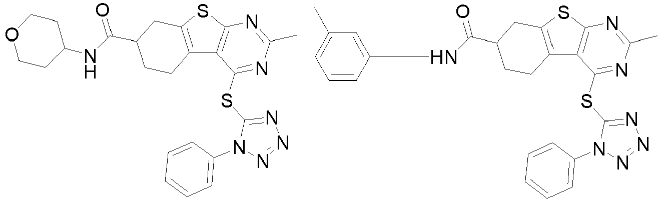

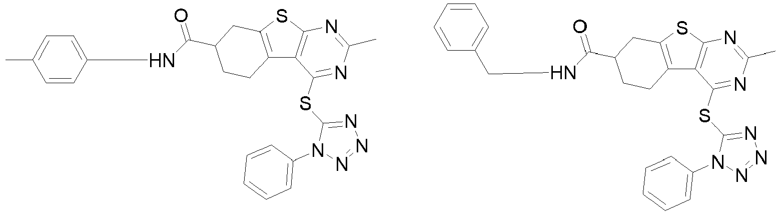

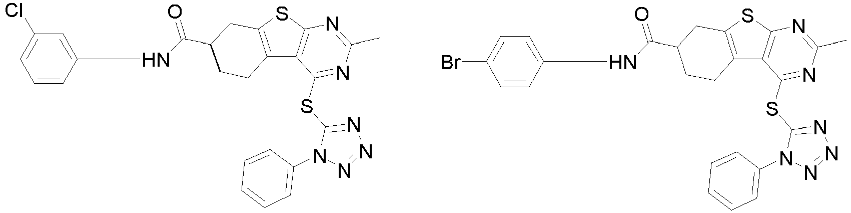

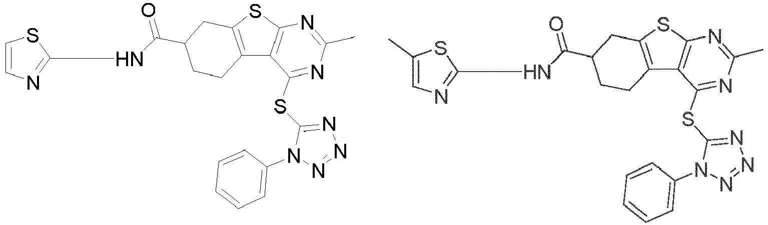

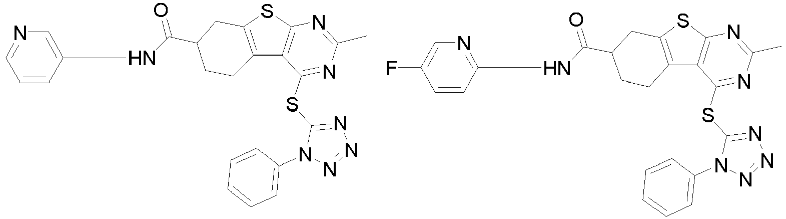

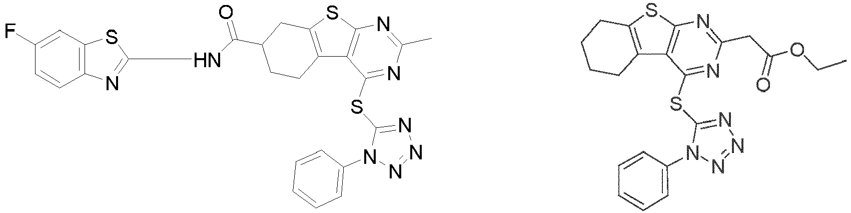

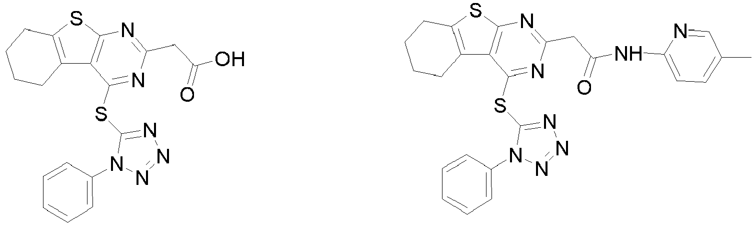

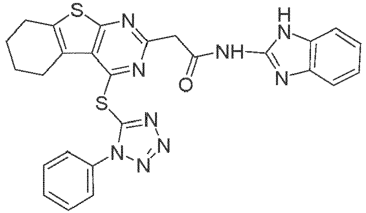

Preferably, the compound for inhibiting RORyt activity is one selected from the group consisting of:

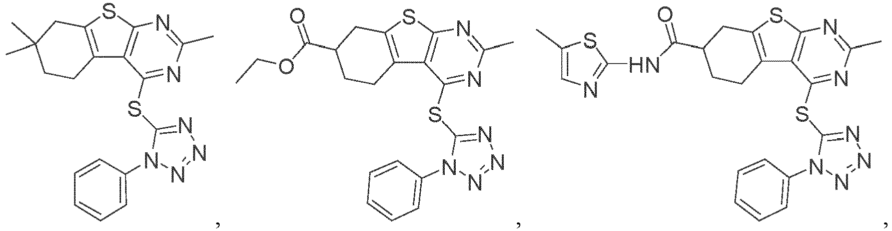

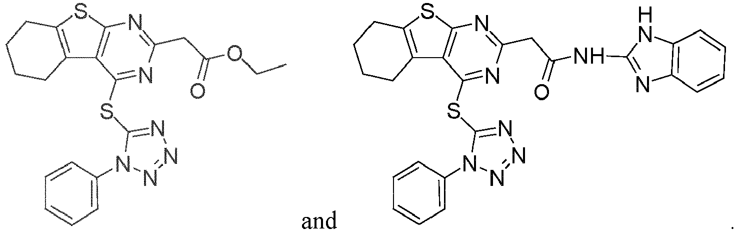

The present disclosure also provides a method for preparing a compound for inhibiting RORyt activity including the following steps: reacting compounds 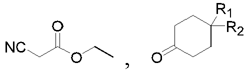 with elemental sulfur in the presence of morpholine and ethanol to prepare a compound 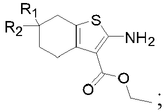 reacting the prepared compound 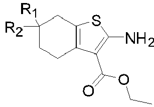 with NC-R₃ in a solution of hydrogen chloride in 1,4-dioxane to prepare a compound 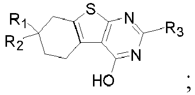 reacting the prepared compound 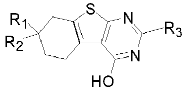 with phosphorus oxychloride to prepare a compound 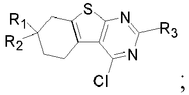 and reacting the prepared compound 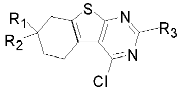 with a compound 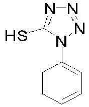 in the presence of sodium bicarbonate and dimethyl sulfoxide to prepare a compound

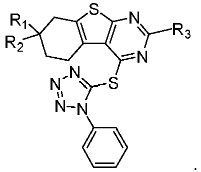

In one specific embodiment, the compound 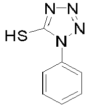 is prepared by reacting phenyl thioisocyanate and sodium azide in an aqueous solution.

More specifically, one of R₁ and R₂ is hydrogen, and the other is 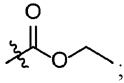 and the preparation method further includes reacting a compound 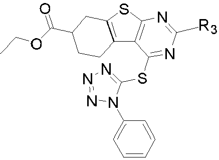 in a lithium hydroxide aqueous solution to prepare a compound

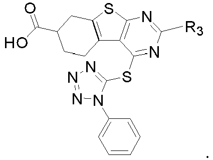

More specifically, one of R₁ and R₂ is hydrogen, and the other is 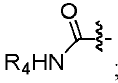 ; and the preparation method further includes reacting the prepared compound 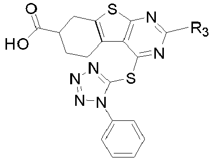 with R₄NH₂, HOBT and EDCI in an ultra-dry DCM solvent to prepare a compound

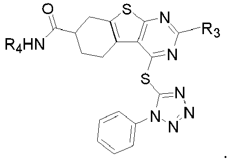

The compound for inhibiting RORyt activity as described above may be used in preparation of a therapeutic formulation for the treatment of autoimmune diseases, in preparation of a formulation that inhibits formation of a Th17 cell, in preparation of a formulation that inhibits the RORyt transcription activity, or in preparation of a formulation that inhibits transcription and expression of IL17A gene and/or IL17F gene. The compounds having the structural formula (A) has a good inhibitory effect on RORyt activity. Specifically, luciferase activity reporter system and Th17 cell differentiation experiments prove that the compound may inhibit the transcription effect of a key transcription factor RORyt for Th7 cell differentiation, and may inhibit the transcription and expression of genes IL17A and IL17F targeted by RORγt. The intracellular expression of IL17A at the protein level is also significantly inhibited as detected by the flow cytometer, indicating that the compound molecule has an obvious inhibitory effect on Th17 cell activity. Therefore, the compound may be used to produce a formulation of corresponding activity inhibitor, and provide a leading structure for the development of therapeutic drugs for autoimmune diseases.

The compound for inhibiting RORyt activity, the preparation method therefor and the application thereof of the present disclosure will be further described in detail below with reference to specific examples.

### I. Preparation of compounds and intermediates

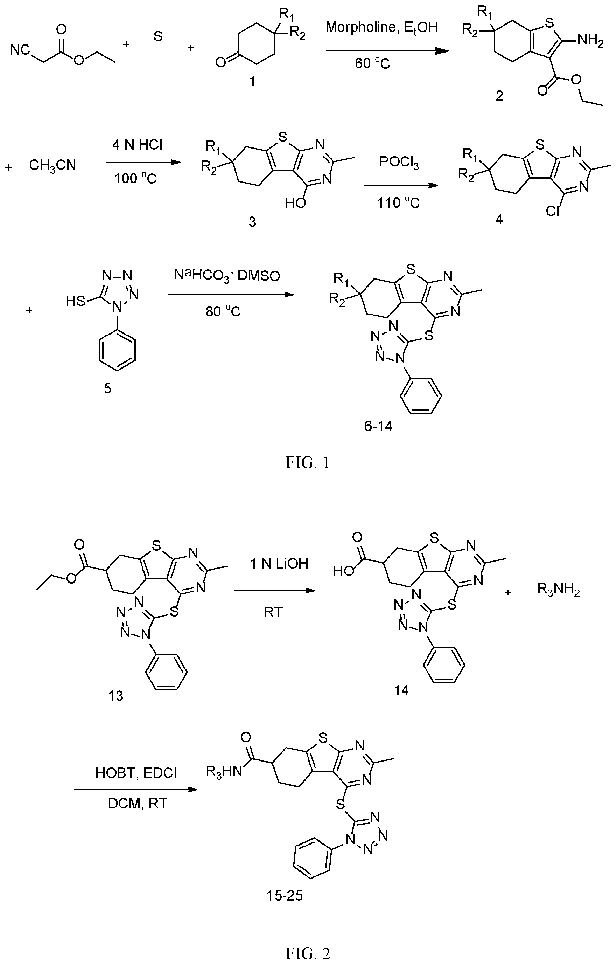

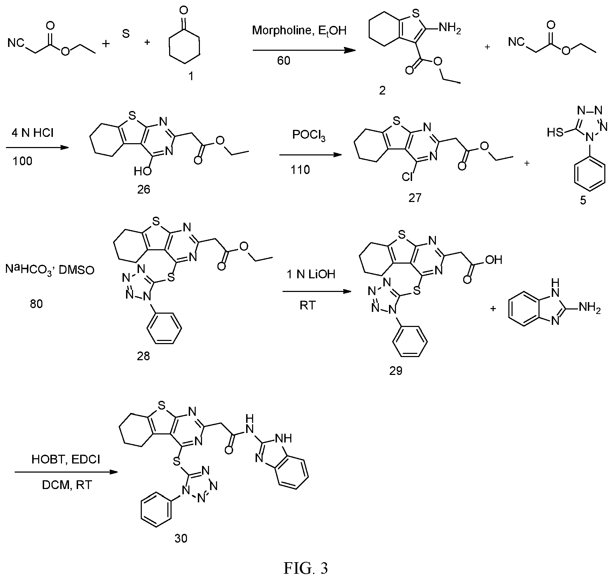

Referring to FIG. 1, FIG. 2, and FIG. 3, the preparation and test results of each compound and intermediate are as follows:

### 1. Intermediate 2

Compound 1 (4-cyclohexanone derivative, 5.50 mmol), morpholine (5.50 mmol), ethyl cyanoacetate (5.01 mmol), elemental sulfur (5.50 mmol), and 5 mL of ethanol were added into a 100 mL evaporating flask. The reaction solution was stirred at 60°C overnight, and cooled to room temperature, and then the solvent was evaporated to dryness by rotary evaporation. The residue was separated and purified (cyclohexane:ethyl acetate=50:1) by column chromatography to obtain an Intermediate 2 (white or yellow solid, 95% yield).

### 2. Intermediate 3

Compound 2 was added into a 50 mL sealed tube and dissolved in 5 mL of acetonitrile, and then 4 mL of a solution of HCl (4 mol/L) in 1,4-dioxane was added. The reaction solution was stirred at 100°C overnight, then cooled to room temperature, and then the solvent was evaporated to dryness by rotary evaporation. The residue was dissolved in an appropriate amount of ethyl acetate and adjusted to be alkaline by adding a saturated aqueous NaHCO₃ solution. Then the mixture was extracted with ethyl acetate, and washed with a saturated NaCl aqueous solution, and the organic layer was evaporated to dryness by rotary evaporation to obtain an Intermediate 3 (white solid, 75% yield), which was directly put into next reaction without purification.

### 3. Intermediate 4

Intermediate 3 (0.82 mmol) and 5 mL of POCl₃ were added into a 100 mL evaporating flask. The reaction solution was refluxed under stirring at 110°C for 3 h, then cooled to room temperature and evaporated to dryness by rotary evaporation under reduced pressure. The residue was dissolved in an appropriate amount of ethyl acetate and adjusted to be alkaline by adding a saturated aqueous NaHCO₃ solution. Then the mixture was extracted with ethyl acetate, washed with a saturated NaCl aqueous solution, and the organic layer was evaporated to dryness by rotary evaporation to obtain an Intermediate 4 (white solid, 97% yield), which was directly put into next reaction without purification.

### 4. Intermediate 5

Phenyl thioisocyanate (2700 mg, 20.00 mmol), NaN₃ (1952 mg, 30.04 mmol), and 20 mL of water were added into a 100 mL evaporating flask. The reaction solution was stirred at 90°C overnight, and then cooled to room temperature. Then the reaction solution was extracted with ethyl acetate to remove by-products in the organic layer. The water layer was acidified with 1 N of HCl aqueous solution, and the mixture was extracted with ethyl acetate, washed with a saturated NaCl aqueous solution, and the organic layer was evaporated to dryness by rotary evaporation to obtain an Intermediate 5 (white solid, 75% yield), which was directly put into next reaction without purification.

### 5. Compound 6

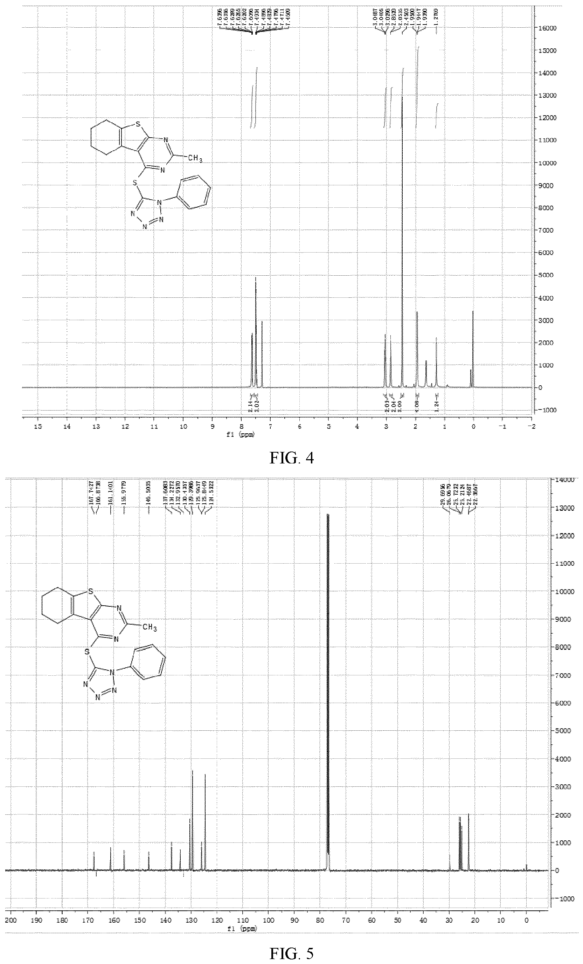

Intermediate 4 (0.65 mmol), Intermediate 5 (0.73 mmol), NaHCO₃ (1.30 mmol), and 5 mL of DMSO were added into a 100 mL evaporating flask. The reaction solution was stirred at 80°C overnight, and then cooled to room temperature. The reaction solution was extracted with ethyl acetate, and washed with a saturated NaCl aqueous solution, and the organic layer was evaporated to dryness by rotary evaporation, then the residue was separated and purified by column chromatography (cyclohexane:ethyl acetate = 25:1) to obtain an Intermediate 6 (white solid, 63% yield). Test data: ¹H NMR spectrum and ¹³C NMR spectrum are shown in FIG. 4 and FIG. 5, respectively. ¹H NMR (500 MHz, CDCl₃) δ 7.63 (dt, *J* = 8.4, 3.6 Hz, 2H), 7.55 - 7.44 (m, 3H), 3.04 (t, *J* = 4.8 Hz, 2H), 2.86 (d, *J* = 5.2 Hz, 2H), 2.45 (s, 3H), 1.99 - 1.88 (m, 4H), 1.28 (s, 1H). ¹³C NMR (126 MHz, CDCl₃) δ 167.74 (s), 161.14 (s), 155.98 (s), 146.50 (s), 137.61 (s), 134.23 (s), 130.44 (s), 129.40 (s), 125.90 (d, *J* = 14.9 Hz), 124.53 (s), 29.70 (s), 26.07 (s), 25.72 (s), 25.21 (s), 22.42 (d, *J* = 12.8 Hz). ESI-MS *m*/*z*: 381.2 [M+H]⁺.

### 6. Compound 7

The experiment was operated with reference to that for Compound 6. Test data: ¹H NMR (500 MHz, DMSO-d₆) δ 7.69 (dd, *J* = 7.7, 1.8 Hz, 2H), 7.58 - 7.44 (m, 3H), 3.13 - 3.00 (m, 1H), 2.90 (dd, *J* = 17.0, 4.5 Hz, 2H), 2.41 (dd, *J* = 17.9, 10.3 Hz, 1H), 2.36 (s, 3H), 1.93 (s, 2H), 1.52 - 1.42 (m, 1H), 1.05 (d, *J* = 6.5 Hz, 3H). ¹³C NMR (126 MHz, DMSO-d₆) δ 167.38 (s), 161.09 (s), 156.58 (s), 147.59 (s), 137.40 (s), 134.03 (s), 131.13 (s), 129.97 (s), 125.96 (s), 125.44 (s), 33.37 (s), 30.28 (s), 28.68 (s), 25.76 (s), 25.36 (s), 21.39 (s). ESI-MS *m*/*z*: 395.10 [M+H]⁺.

### 7. Compound 8

The experiment was operated with reference to that for Compound 6. Test data: ¹H NMR (500 MHz, CDCl₃) δ 7.64 - 7.57 (m, 2H), 7.50 - 7.42 (m, 3H), 3.17 (dd, *J* = 10.6, 8.2 Hz, 1H), 2.98 - 2.86 (m, 2H), 2.49 - 2.43 (m, 1H), 2.42 (s, 3H), 2.12 - 2.04 (m, 1H), 1.82 - 1.70 (m, 1H), 1.55 - 1.38 (m, 3H), 0.99 (t, *J* = 7.4 Hz, 3H). ¹³C NMR (126 MHz, CDCl₃) δ 167.90 (s), 161.13 (s), 155.98 (s), 146.52 (s), 137.45 (s), 134.21 (s), 130.47 (s), 129.43 (s), 125.89 (s), 124.55 (s), 35.62 (s), 31.66 (s), 28.40 (d, *J* = 20.6 Hz), 28.31 - 28.13 (m), 25.85 (s), 25.24 (s), 11.44 (s). ESI-MS *m*/*z*: 409.26 [M+H]⁺.

### 8. Compound 9

The experiment was operated with reference to that for Compound 6. Test data: ¹H NMR (500 MHz, DMSO-d₆) δ 7.69 (dd, *J* = 7.6, 1.9 Hz, 2H), 7.56 - 7.46 (m, 3H), 3.07 (d, *J* = 16.0 Hz, 1H), 2.95 - 2.82 (m, 2H), 2.42 (dd, *J* = 17.2, 9.9 Hz, 1H), 2.35 (s, 3H), 1.99 (d, *J* = 13.5 Hz, 1H), 1.80 (s, 1H), 1.46 (ddd, *J* = 23.8, 10.8, 5.8 Hz, 1H), 1.41 - 1.29 (m, 4H), 0.90 (t, *J* = 6.8 Hz, 3H). ¹³C NMR (126 MHz, DMSO-d₆) δ 167.40 (s), 161.08 (s), 156.54 (s), 147.58 (s), 137.54 (s), 134.03 (s), 131.13 (s), 129.97 (s), 126.19 (s), 125.44 (s), 37.83 (s), 33.33 (s), 31.67 (s), 28.46 (s), 25.77 (s), 25.37 (s), 19.90 (s), 14.61 (s). ESI-MS *m*/*z*: 423.21 [M+H]⁺.

### 9. Compound 10

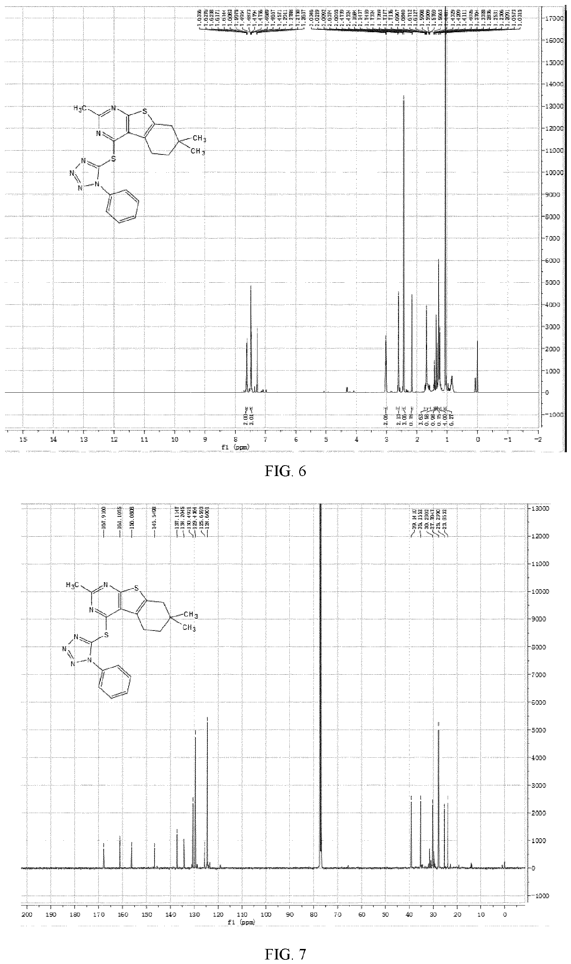

The experiment was operated with reference to that for Compound 6. Test data: ¹H NMR spectrum and ¹³C NMR spectrum are shown in FIG. 6 and FIG. 7, respectively. ¹H NMR (500 MHz, CDCl₃) δ 7.65 - 7.59 (m, 2H), 7.51 - 7.44 (m, 3H), 3.02 (t, J = 6.4 Hz, 2H), 2.62 (d, J = 15.9 Hz, 2H), 2.45 (d, J = 20.8 Hz, 3H), 2.16 (d, J = 12.3 Hz, 1H), 1.76 - 1.54 (m, 4H), 1.44 - 1.40 (m, 1H), 1.37 (s, 1H), 1.33 (s, 1H), 1.24 (dd, J = 24.2, 13.0 Hz, 4H), 1.06 (s, 6H). ¹³C NMR (126 MHz, CDCl₃) δ 167.92 (s), 161.11 (s), 156.08 (s), 146.55 (s), 137.11 (s), 134.20 (s), 130.49 (s), 129.44 (s), 125.66 (s), 124.60 (s), 39.14 (s), 35.16 (s), 31.48 (d, J = 8.8 Hz), 30.51 - 29.31 (m), 29.39 - 29.31 (m), 27.70 (s), 25.24 (s), 23.85 (s). ESI-MS *m*/*z*: 409.15 [M+H]⁺.

### 10. Compound 11

The experiment was operated with reference to that for Compound 6. Test data: ¹H NMR (500 MHz, CDCl₃) δ 7.60 (dd, *J* = 6.4, 3.0 Hz, 2H), 7.45 (dd, *J* = 7.2, 3.6 Hz, 3H), 3.24 (dd, *J* = 16.1, 4.9 Hz, 1H), 2.85 (td, *J* = 16.5, 5.2 Hz, 2H), 2.62 - 2.53 (m, 1H), 2.42 (s, 3H), 2.18 - 2.13 (m, 1H), 1.58 (ddd, *J* = 11.8, 4.5, 3.0 Hz, 1H), 1.43 (ddd, *J=* 24.7, 12.3, 5.1 Hz, 1H), 0.96 (s, 9H). ¹³C NMR (126 MHz, CDCl₃) δ 167.95 (s), 161.12 (s), 155.89 (s), 146.50 (s), 138.40 (s), 134.18 (s), 130.48 (s), 129.43 (s), 125.93 (s), 125.57 (s), 124.52 (s), 44.52 (s), 32.51 (s), 27.22 (t, J = 18.6 Hz), 25.27 (s), 24.05 (s). ESI-MS *m*/*z*: 437.26 [M+H]⁺.

### 11. Compound 12

The experiment was operated with reference to that for Compound 6. Test data: ¹H NMR (500 MHz, CDCl₃) δ 7.63 - 7.57 (m, 2H), 7.51 - 7.43 (m, 3H), 3.34 (ddd, *J* = 30.6, 12.3, 4.6 Hz, 4H), 2.36 (ddd, *J* = 20.1, 13.4, 6.6 Hz, 2H). ¹³C NMR (126 MHz, DMSO-d₆) δ 167.97 (s), 161.71 (s), 157.22 (s), 147.50 (s), 133.95 (s), 131.13 (s), 129.97 (s), 125.41 (s), 124.77 (s), 124.52 (s), 34.97 (d, *J* = 28.7 Hz), 34.76 - 34.65 (m), 34.62 (s), 29.94 (s), 29.65 (d, *J* = 24.2 Hz), 29.41 - 29.18 (m), 25.39 (s), 23.86 (s). ESI-MS *m*/*z*: 417.14 [M+H]⁺.

### 12. Compound 13

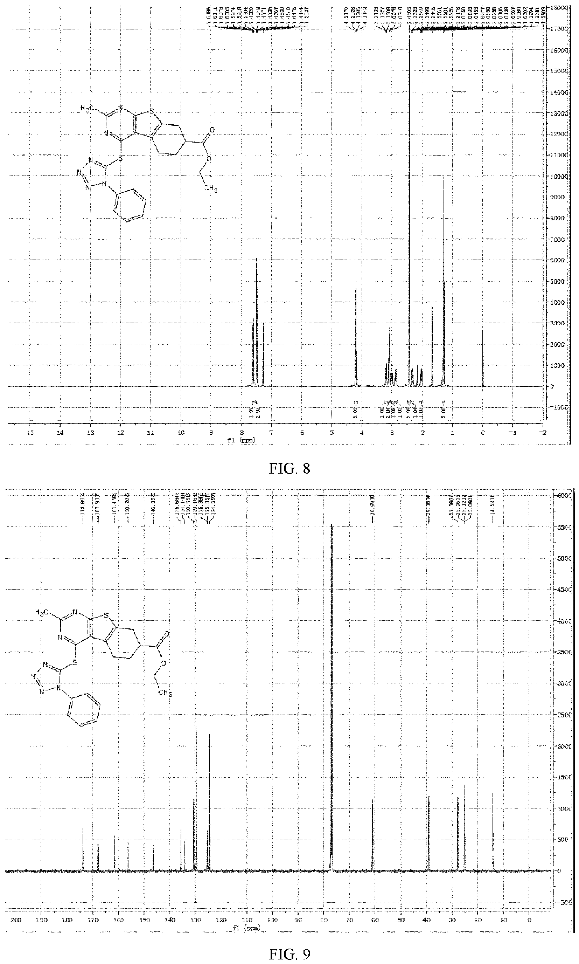

The experiment was operated with reference to that for Compound 6. Test data: ¹H NMR spectrum and ¹³C NMR spectrum are shown in FIG. 8 and FIG. 9, respectively. ¹H NMR (500 MHz, CDCl₃) δ 7.63 - 7.57 (m, 2H), 7.52 - 7.44 (m, 3H), 4.20 (q, J = 7.1 Hz, 2H), 3.20 (ddd, J = 16.3, 5.3, 4.4 Hz, 1H), 3.09 (d, J = 6.5 Hz, 2H), 3.07 - 2.96 (m, 1H), 2.92 - 2.80 (m, 1H), 2.41 (d, J = 15.8 Hz, 3H), 2.39 - 2.28 (m, 1H), 2.08 - 1.97 (m, 1H), 1.29 (t, J = 7.1 Hz, 3H). ¹³C NMR (126 MHz, CDCl₃) δ 173.90 (s), 167.91 (s), 161.48 (s), 156.26 (s), 146.33 (s), 135.68 (s), 134.15 (s), 130.53 (s), 129.46 (s), 125.36 (d, J = 7.5 Hz), 124.56 (s), 60.99 (s), 39.17 (s), 27.79 (s), 25.54 - 24.89 (m), 14.23 (s). ESI-MS *m*/*z*: 453.26 [M+H]⁺.

### 13. Compound 14

Compound 13 (1.30 mmol) was added into a 100 mL evaporating flask and dissolved in 10 ml of ultra-dry THF solvent, and 1 N of LiOH aqueous solution (3.9 ml, 3.9 mmol) was added. The reaction solution was stirred at room temperature overnight, then acidified by adding 1 mL of HCl aqueous solution, then extracted with ethyl acetate, and washed with a saturated NaCl aqueous solution, and the organic layer was evaporated to dryness by rotary evaporation, then the residue was separated and purified by column chromatography (cyclohexane:acetone = 3:1) to obtain an Intermediate 14 (white or yellow solid, 49% yield). Test data: ¹H NMR (500 MHz, DMSO-d₆) δ 12.49 (s, 1H), 7.69 (dd, *J* = 7.9, 1.7 Hz, 2H), 7.54 - 7.45 (m, 3H), 3.10 - 3.01 (m, 2H), 2.94 (dd, *J=* 16.9, 8.6 Hz, 2H), 2.88 - 2.78 (m, 1H), 2.35 (s, 3H), 2.24 - 2.15 (m, 1H), 1.88 (ddd, *J=* 15.5, 12.5, 7.6 Hz, 1H). ¹³C NMR (126 MHz, DMSO-d₆) δ 175.67 (s), 167.42 (s), 161.25 (s), 156.66 (s), 147.55 (s), 136.37 (s), 134.00 (s), 131.13 (s), 129.98 (s), 125.80 (s), 125.32 (d, *J* = 27.1 Hz), 38.61 (s), 27.73 (s), 25.24 (d, *J* = 31.8 Hz), 25.00 (s). ESI-MS *m*/*z*: 423.17 [M-H]⁻.

### 14. Compound 15

Compound 14 (0.28 mmol), a heteroaromatic amine derivative (0.57 mmol), HOBT (0.31 mmol), and EDCI (0.31 mmol) were added into a 100 mL evaporating flask and dissolved in 10 mL of ultra-dry DCM solvent. The reaction solution was stirred at room temperature overnight, then evaporated to dryness by rotary evaporation under reduced pressure. The residue was extracted with ethyl acetate, washed with a saturated NaCl aqueous solution, and the organic layer was evaporated to dryness by rotary evaporation, then the residue was separated and purified by column chromatography (cyclohexane:acetone = 10:1) to obtain an Intermediate 15 (yellow solid, 63% yield). Test data: ¹H NMR (500 MHz, DMSO-d₆) δ 8.17 (d, J = 8.4 Hz, 1H), 8.01 (d, J = 7.5 Hz, 1H), 7.96 (d, J = 7.5 Hz, 1H), 7.84 (d, J = 8.3 Hz, 1H), 7.72 - 7.67 (m, 2H), 7.63 (d, J = 8.0 Hz, 1H), 7.51 (d, J = 6.9 Hz, 3H), 3.81 (d, J = 11.0 Hz, 3H), 3.78 - 3.72 (m, 1H), 3.10 (d, J = 14.9 Hz, 1H), 3.02 (d, J = 5.6 Hz, 1H), 2.91 (s, 3H), 2.72 (d, J = 8.3 Hz, 1H), 2.62 (d, J = 6.0 Hz, 1H), 2.35 (s, 3H), 2.30 (s, 1H), 2.08 (d, J = 10.8 Hz, 2H), 1.80 (d, J = 7.3 Hz, 2H), 1.69 (s, 3H), 1.39 (dd, J = 15.6, 7.2 Hz, 3H), 1.20 (s, 1H). ¹³C NMR (126 MHz, DMSO-d₆) δ 173.16 (s), 167.40 (s), 161.18 (s), 156.60 (s), 147.63 (s), 136.86 (s), 133.98 (s), 131.15 (s), 129.98 (s), 125.69 (s), 125.44 (s), 125.27 (s), 66.35 (s), 45.36 (s), 32.95 (d, J = 8.0 Hz), 28.31 (s), 26.12 (s), 25.43 (d, J = 15.8 Hz). ESI-MS *m*/*z*: 530.23 [M+Na] ⁺.

### 15. Compound 16

The experiment was operated with reference to that for Compound 15; Yield = 66%. Test data: ¹H NMR (500 MHz, DMSO-d₆) δ 9.98 (s, 1H), 7.70 (dd, *J* = 7.7, 1.8 Hz, 2H), 7.56 - 7.49 (m, 3H), 7.47 (s, 1H), 7.39 (d, *J* = 8.2 Hz, 1H), 7.17 (t, *J* = 7.8 Hz, 1H), 6.85 (d, *J* = 7.5 Hz, 1H), 3.16 (d, *J* = 15.7 Hz, 1H), 3.09 - 2.84 (m, 4H), 2.37 (s, 3H), 2.26 (s, 3H), 2.25 - 2.19 (m, 1H), 1.96 - 1.85 (m, 1H). ¹³C NMR (126 MHz, DMSO-d₆) δ 172.87 (s), 167.46 (s), 161.23 (s), 157.27 (s), 156.63 (s), 147.62 (s), 139.57 (s), 138.34 (s), 136.68 (s), 134.02 (s), 131.14 (s), 129.98 (s), 129.01 (s), 125.73 (s), 125.48 (t, *J* = 25.0 Hz), 124.38 (s), 120.22 (s), 116.86 (s), 28.22 (s), 26.81 (s), 26.08 (s), 25.45 (d, *J* = 18.5 Hz), 23.76 (s), 21.66 (s). ESI-MS *m*/*z*: 514.26 [M+H]⁺.

### 16. Compound 17

The experiment was operated with reference to that for Compound 15; Yield = 70%. ¹H NMR (500 MHz, DMSO-d₆) δ 9.97 (s, 1H), 7.74 - 7.66 (m, 2H), 7.52 (dd, *J* = 9.6, 2.6 Hz, 5H), 7.16 (d, *J* = 8.5 Hz, 2H), 3.16 (d, *J* = 15.9 Hz, 1H), 3.03 (dd, *J* = 16.0, 7.3 Hz, 2H), 2.96 (t, *J* = 8.9 Hz, 1H), 2.90 - 2.79 (m, 2H), 2.37 (s, 3H), 2.22 (d, *J* = 13.3 Hz, 1H), 1.95 - 1.85 (m, 1H), 1.16 (d, *J* = 6.9 Hz, 6H). ¹³C NMR (126 MHz, DMSO-d₆) δ 172.70 (s), 167.47 (s), 161.23 (s), 156.62 (s), 147.64 (s), 143.76 (s), 137.41 (s), 136.72 (s), 134.01 (s), 131.14 (s), 129.98 (s), 126.87 (s), 125.71 (s), 125.38 (d, *J* = 16.7 Hz), 119.77 (s), 33.33 (s), 28.26 (s), 26.12 (s), 25.92 - 25.66 (m), 25.46 (d, *J* = 21.2 Hz), 24.43 (s). ESI-MS *m*/*z*: 542.35 [M+H]⁺.

### 17. Compound 18

The experiment was operated with reference to that for Compound 15; Yield = 44%. ¹H NMR (500 MHz, DMSO-d₆) δ 8.50 (t, *J* = 5.9 Hz, 1H), 7.74 - 7.64 (m, 2H), 7.56 - 7.47 (m, 3H), 7.31 (t, *J* = 7.4 Hz, 2H), 7.28 - 7.19 (m, 3H), 4.33 - 4.28 (m, 2H), 3.10 (s, 1H), 2.95 (dd, *J* = 29.7, 23.5 Hz, 3H), 2.81- 2.70 (m, 1H), 2.36 (s, 3H), 2.19 - 2.10 (m, 1H), 1.96 - 1.78 (m, 1H). ¹³CNMR (126 MHz, DMSO-d₆) δ 129.98 (s), 128.78 (s), 127.59 (s), 125.36 (d, *J* = 18.6 Hz), 41.40 - 39.92 (m), 39.74 (d, *J* = 21.0 Hz), 39.49 (s), 25.42 (d, *J* = 12.2 Hz). ESI-MS *m*/*z*: 514.17 [M+H]⁺.

### 18. Compound 19

The experiment was operated with reference to that for Compound 15; Yield = 32%. ¹H NMR (500 MHz, CDCl₃) δ 8.36 (s, 1H), 7.83 (s, 1H), 7.61 - 7.55 (m, 2H), 7.49 (dd, *J* = 14.4, 7.9 Hz, 4H), 7.24 (d, *J* = 8.1 Hz, 1H), 7.09 (d, *J* = 7.9 Hz, 1H), 3.20 (dd, *J* = 15.8, 7.3 Hz, 2H), 3.06 - 2.97 (m, 2H), 2.94 (d, *J* = 11.1 Hz, 1H), 2.41 (s, 3H), 2.36 (d, *J* = 6.0 Hz, 1H), 2.06 (dd, *J* = 12.6, 5.4 Hz, 1H). ¹³C NMR (126 MHz, CDCl₃) δ 172.55 (s), 167.89 (s), 161.41 (s), 155.72 (s), 146.52 (s), 139.19 (s), 136.08 (s), 134.70 (s), 133.92 (s), 130.76 (s), 130.00 (s), 129.58 (s), 125.14 (s), 124.93 (s), 124.48 (s), 120.04 (s), 117.84 (s), 41.76 (s), 28.22 (s), 26.92 (s), 26.27 (s), 25.35 (d, *J* = 14.7 Hz). ESI-MS *m*/*z:* 534.20 [M+H]⁺.

### 19. Compound 20

The experiment was operated with reference to that for Compound 15; Yield = 49%. ¹H NMR (500 MHz, DMSO-d₆) δ 10.20 (s, 1H), 7.72 - 7.66 (m, 2H), 7.60 (d, *J* = 8.9 Hz, 2H), 7.55 - 7.44 (m, 5H), 5.47 (d, *J* = 7.5 Hz, 2H), 3.62 (dq, *J* = 13.1, 6.5 Hz, 2H), 3.16 (d, *J* = 16.1 Hz, 1H), 3.08 - 2.84 (m, 4H), 2.37 (s, 3H), 2.23 (dd, *J* = 10.8, 2.4 Hz, 1H), 1.95 - 1.85 (m, 1H). ¹³C NMR (126 MHz, DMSO-d₆) δ 173.13 (s), 167.47 (s), 161.25 (s), 157.27 (s), 156.65 (s), 147.63 (s), 139.00 (s), 136.56 (s), 134.01 (s), 132.01 (s), 131.15 (s), 129.98 (s), 125.70 (s), 125.45 (s), 121.58 (s), 115.25 (s), 28.16 (s), 25.99 (s), 25.44 (d, *J* = 15.3 Hz), 23.76 (s). ESI-MS *m*/*z*: 578.09 [M-H] ⁻.

### 20. Compound 21

The experiment was operated with reference to that for Compound 15; Yield = 43%. ¹H NMR (500 MHz, DMSO-d₆) δ 12.28 (s, 1H), 7.69 (dd, *J* = 7.7, 1.8 Hz, 2H), 7.55 - 7.50 (m, 3H), 7.48 (d, *J* = 3.5 Hz, 1H), 7.22 (d, *J* = 3.5 Hz, 1H), 3.18 - 3.02 (m, 4H), 2.94 (dd, *J* = 13.8, 6.4 Hz, 1H), 2.37 (s, 3H), 2.24 (d, *J* = 13.3 Hz, 1H), 1.98 - 1.89 (m, 1H). ¹³C NMR (126 MHz, DMSO-d₆) δ 172.90 (s), 167.45 (s), 161.30 (s), 158.40 (s), 156.67 (s), 147.63 (s), 138.13 (s), 136.31 (s), 134.00 (s), 131.15 (s), 129.99 (s), 125.71 (s), 125.43 (s), 125.26 (s), 114.02 (s), 31.16 (s), 27.77 (s), 25.81 (s), 25.38 (s). ESI-MS *m*/*z*: 507.19 [M+H]⁺.

### 21. Compound 22

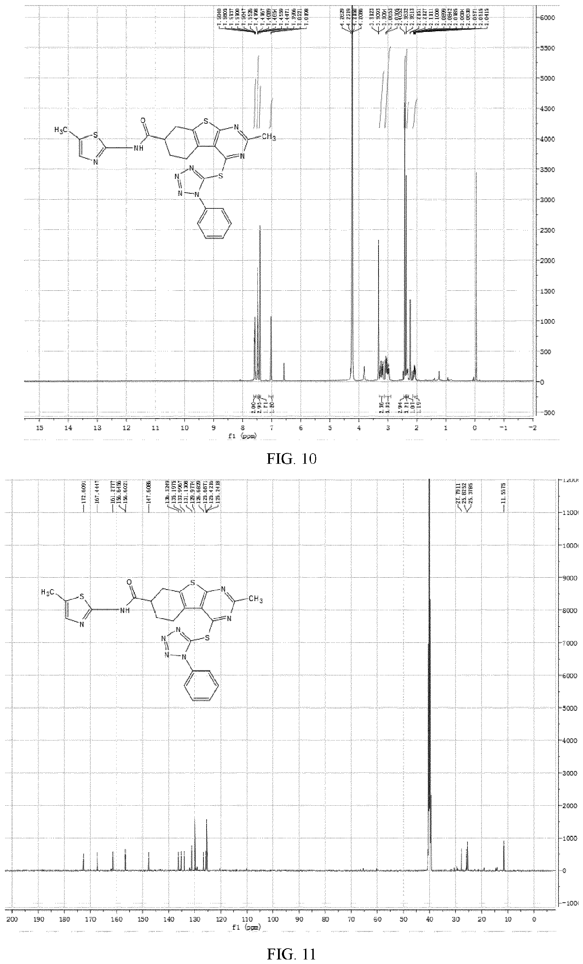

The experiment was operated with reference to that for Compound 15; Yield = 20%; ¹H NMR spectrum and ¹³C NMR spectrum are shown in FIG. 10 and FIG. 11, respectively. ¹H NMR (500 MHz, MeOD) δ 7.57 (dt, J = 8.7, 3.9 Hz, 2H), 7.51 - 7.43 (m, 3H), 7.40 (s, 2H), 7.02 (d, J = 1.1 Hz, 1H), 3.28 - 3.11 (m, 2H), 3.01 (ddd, J = 30.5, 19.2, 7.3 Hz, 3H), 2.41 (s, 3H), 2.36 (d, J = 0.9 Hz, 3H), 2.30 (dd, J = 16.7, 10.2 Hz, 1H), 2.13 - 1.99 (m, 1H). ¹³C NMR (126 MHz, MeOD) δ 133.85 (s), 130.74 (s), 129.53 (s), 124.49 (s), 77.54 (s), 77.40 (d, J = 32.2 Hz), 77.01 (s), 49.18 (s), 49.09 - 48.65 (m), 48.50 (s), 48.33 (s), 48.15 (s), 40.09 (s), 27.73 (s), 25.77 (s), 25.21 (s), 24.81 (s), 11.20 (s). ESI-MS *m*/*z*: 521.25 [M+H]⁺.

### 22. Compound 23

The experiment was operated with reference to that for Compound 15; Yield = 38%. ¹H NMR (500 MHz, DMSO-d₆) δ 10.31 (s, 1H), 8.78 (d, *J* = 2.3 Hz, 1H), 8.27 (dd, *J* = 4.6, 1.3 Hz, 1H), 8.08 (ddd, *J* = 8.3, 2.3, 1.5 Hz, 1H), 7.83 - 7.66 (m, 2H), 7.62 - 7.42 (m, 2H), 7.36 (dd, *J* = 8.2, 4.7 Hz, 1H), 3.26 - 2.81 (m, 5H), 2.60 -2.45 (m, 4H), 2.39 (s, 3H), 2.27 (dd, *J* = 8.9, 5.0 Hz, 1H), 2.10 (d, *J* = 16.4 Hz, 1H), 2.03 - 1.84 (m, 1H). ¹³CNMR (126 MHz, DMSO-d₆) δ 173.71 (s), 167.42 (s), 161.25 (s), 156.66 (s), 147.60 (s), 143.27 (s), 139.63 (s), 136.76 (s), 136.43 (s), 133.98 (s), 131.16 (s), 129.98 (s), 128.16 (s), 125.69 (s), 125.44 (s), 125.24 (s), 124.85 (s), 28.00 (s), 25.93 (s), 25.39 (d, *J* = 4.8 Hz). ESI-MS *m*/*z*: 501.36 [M+H]⁺.

### 23. Compound 24

The experiment was operated with reference to that for Compound 15; Yield = 33%. ¹H NMR (500 MHz, DMSO-d₆) δ 8.34 (d, J = 3.0 Hz, 1H), 8.16 (dd, J = 9.1, 4.1 Hz, 1H), 7.81 - 7.63 (m, 3H), 7.61 -7.46 (m, 3H), 3.14 (t, J = 16.1 Hz, 1H), 3.12 - 2.85 (m, 4H), 2.38 (s, 3H), 2.31 - 2.15 (m, 1H), 1.90 (d, J = 7.9 Hz, 1H). ¹³C NMR (126 MHz, DMSO-d₆) δ 173.69 (s), 167.43 (s), 161.25 (s), 156.63 (s), 149.02 (s), 147.63 (s), 136.60 (s), 135.88 (s), 135.68 (s), 133.98 (s), 131.15 (s), 129.99 (s), 125.76 (d, *J* = 19.8 Hz), 125.44 (s), 125.25 (s), 115.03 (d, *J* = 3.5 Hz), 27.97 (s), 26.12 (s), 25.46 (d, *J* = 19.1 Hz). ESI-MS *m*/*z*: 519.21 [M+H]⁺.

### 24. Compound 25

The experiment was operated with reference to that for Compound 15; Yield = 42%. ¹H NMR (600 MHz, DMSO-d₆) δ 12.59 (s, 1H), 7.93 - 7.87 (m, 1H), 7.76 (dt, *J* = 8.9, 4.4 Hz, 1H), 7.74 - 7.69 (m, 2H), 7.53 (t, *J* = 1.8 Hz, 3H), 7.29 (ddd, *J* = 9.2, 7.5, 3.0 Hz, 1H), 3.21 - 3.02 (m, 4H), 2.98 (dd, *J* = 16.4, 8.0 Hz, 1H), 2.45 (s, 1H), 2.39 (s, 3H), 2.36 - 2.27 (m, 1H), 2.09 (s, 1H), 2.02 - 1.93 (m, 1H), 1.28 - 1.17 (m, 1H). ¹³C NMR (126 MHz, DMSO-d₆) δ 174.01 (s), 167.41 (s), 161.27 (s), 160.03 (s), 158.34 (s), 158.12 (s), 156.63 (s), 147.59 (s), 145.64 (s), 136.12 (s), 133.99 (s), 133.14 (d, *J* = 11.0 Hz), 131.13 (s), 129.97 (s), 125.63 (s), 125.42 (s), 125.16 (s), 122.04 (d, *J* = 8.9 Hz), 114.76 (s), 114.56 (s), 108.96 - 108.71 (m), 108.57 (d, *J* = 26.9 Hz), 27.60 (s), 25.69 (s), 25.37 (s). APCI-MS *m*/*z*: 574.8 [M+H]⁺.

### 25. Intermediate 26

Intermediate 2 was added into a 50 mL sealed tube and dissolved in 5 mL of ethyl cyanoacetate, then 4 mL of a solution of HCl (4 mmol/L) in 1,4-dioxane was added. The reaction solution was stirred at 100°C overnight, then cooled to room temperature, and evaporated to dryness by rotary evaporation under reduced pressure. The residue was dissolved in an appropriate amount of ethyl acetate and adjusted to be alkaline by adding a saturated aqueous NaHCO₃ solution. Then the mixed solution was extracted with ethyl acetate, washed with a saturated salt solution, and the organic layer was evaporated to dryness by rotary evaporation under reduced pressure to obtain a crude product of Intermediate 26 (yellow solid, 75% yield), which was directly put into next reaction without purification.

### 26. Intermediate 27

Intermediate 26 (0.82 mmol) and 5 mL of POCl₃ were added into a 100 mL evaporating flask. The reaction solution was refluxed under stirring at 110°C for 3 h, then cooled to room temperature and evaporated to dryness by rotary evaporation under reduced pressure. The residue was dissolved in an appropriate amount of ethyl acetate and adjusted to be alkaline by adding a saturated aqueous NaHCO₃ solution. Then the mixed solution was extracted with ethyl acetate, washed with a saturated salt solution, and the organic layer was evaporated to dryness by rotary evaporation to obtain a crude product of Intermediate 27 (yellow solid, 97% yield), which was directly put into next reaction without purification.

### 27. Compound 28

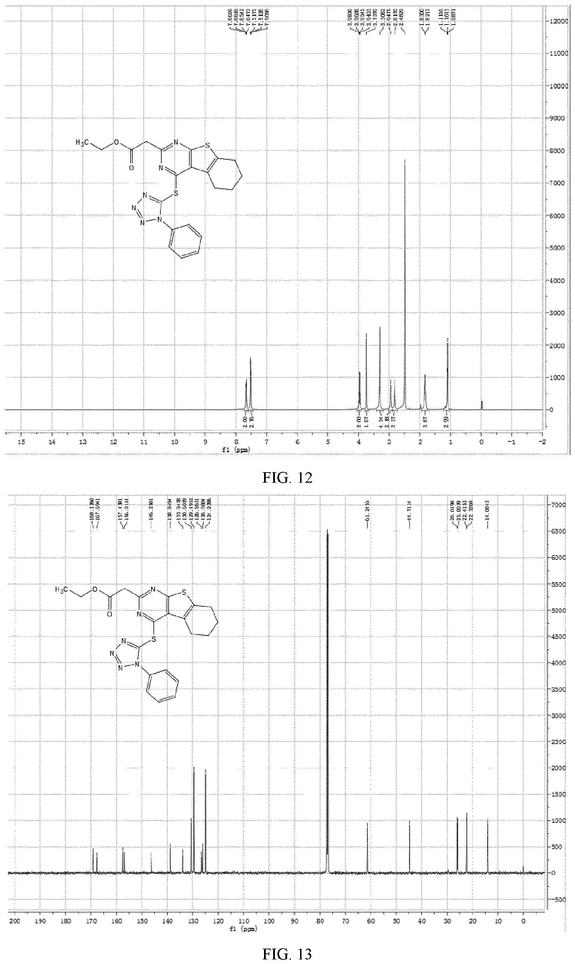

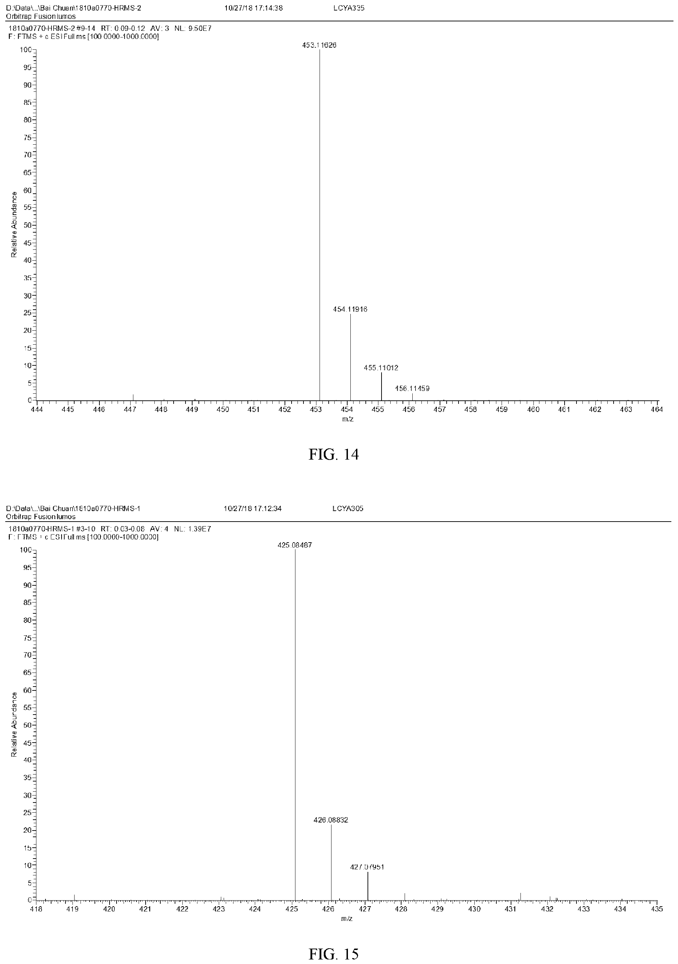

Intermediate 27 (0.65 mmol), Intermediate 5 (0.73 mmol), NaHCO₃ (1.30 mmol), and 5 mL of DMSO were added into a 100 mL evaporating flask. The reaction solution was stirred at 80°C overnight, and then cooled to room temperature. Then the reaction solution was extracted with ethyl acetate, washed with a saturated salt solution, and the organic layer was evaporated to dryness by rotary evaporation, then the residue was separated and purified by column chromatography (cyclohexane:ethyl acetate = 25:1) to obtain a Compound 28 (white solid, 63% yield). Test result: ¹H NMR spectrum and ¹³C NMR spectrum are shown in FIG. 12 and FIG. 13, respectively. ¹H NMR (500 MHz, DMSO-d₆) δ 7.66 (dd, *J* = 6.5, 2.8 Hz, 2H), 7.57 - 7.44 (m, 3H), 3.96 (q, *J=* 7.1 Hz, 2H), 3.74 (s, 2H), 3.31 (s, 5H), 2.95 (s, 2H), 2.82 (s, 2H), 1.83 (d, *J=* 4.4 Hz, 4H), 1.10 (t, *J=* 7.1 Hz, 3H). ¹³C NMR (126 MHz, CDCl₃) δ 169.13 (s), 167.69 (s), 157.44 (s), 156.91 (s), 146.25 (s), 138.85 (s), 133.94 (s), 130.60 (s), 129.49 (s), 126.56 (s), 126.08 (s), 124.93 (s), 61.25 (s), 44.71 (s), 25.91 (d, *J* = 27.1 Hz), 25.75 - 25.61 (m), 22.37 (d, *J* = 11.1 Hz), 14.08 (s). ESI-MS *m*/*z*: 453.11626 [M+H]⁺. HR-ESI-MS spectrum was shown in FIG. 14.

### 28. Compound 29

Compound 28 (1.30 mmol) was added into a 100 mL evaporating flask and dissolved in 10 ml of ultra-dry THF solvent, and 1 N of LiOH aqueous solution (3.9 ml, 3.9 mmol) was added. The reaction solution was stirred at room temperature overnight, then acidified by adding 1 N of HCl aqueous solution, extracted with ethyl acetate, and washed with a saturated salt solution, and the organic layer was evaporated to dryness by rotary evaporation under reduced pressure, then the residue was separated and purified by column chromatography (cyclohexane:acetone = 3:1) to obtain a Compound 29 (yellow solid, 66% yield). Test result: ¹H NMR (500 MHz, DMSO-d₆) δ 7.64 (d, *J* = 7.4 Hz, 2H), 7.44 (dt, *J* = 14.0, 7.0 Hz, 3H), 3.46 (s, 3H), 2.92 (s, 2H), 2.78 (s, 2H), 1.91 - 1.74 (m, 4H). ¹³C NMR (126 MHz, DMSO-d₆) δ 167.09 (s), 156.53 (s), 147.63 (s), 137.90 (s), 133.81 (s), 131.11 (s), 129.99 (s), 127.11 - 126.28 (m), 126.21 (s), 126.17 - 125.23 (m), 26.04 (s), 25.57 (s), 22.31 (d, *J=* 11.7 Hz). ESI-MS *m*/*z*: 425.08487 [M+H] ⁺. HR-ESI-MS spectrum was shown in FIG. 15.

### 29. Compound 30

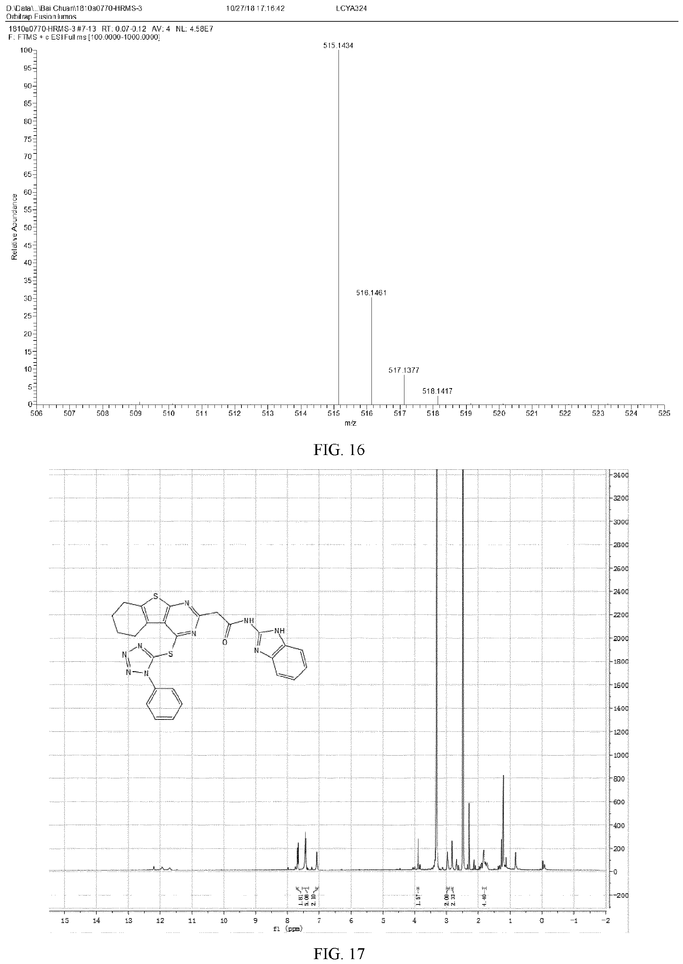

Compound 29 (0.28 mmol), 2-amino-5-methylpyridine (0.57 mmol), HOBT (0.31 mmol), and EDCI (0.31 mmol) were added into a 100 mL evaporating flask and dissolved in 10 mL of ultra-dry DCM solvent. The reaction solution was stirred at room temperature overnight, and then evaporated to dryness by rotary evaporation. The residue was extracted with ethyl acetate, washed with a saturated NaCl aqueous solution, and the organic layer was evaporated to dryness by rotary evaporation, then the residue was separated and purified by column chromatography (cyclohexane:acetone = 10:1) to obtain a Compound 30 (yellow solid, 59% yield). Test result: ¹H NMR (500 MHz, DMSO-d₆) δ 8.14 (d, *J=* 1.3 Hz, 1H), 7.92 - 7.82 (m, 1H), 7.66 (d, *J* = 7.6 Hz, 2H), 7.57 (dd, *J=* 8.5, 2.0 Hz, 1H), 7.47 (d, *J=* 7.4 Hz, 1H), 7.41 (t, *J=* 7.6 Hz, 2H), 3.83 (s, 2H), 2.95 (s, 2H), 2.82 (s, 3H), 2.73 - 2.65 (m, 1H), 2.29 (s, 1H), 2.23 (s, 3H), 1.83 (d, J = 4.9 Hz, 4H). ¹³C NMR (126 MHz, DMSO-d₆) δ 163.55 (s), 158.95 (s), 154.66 (s), 131.20 (s), 130.96 (s), 120.62 (s), 25.73 (s), 24.82 (s), 22.99 (s), 22.26 (s), 21.28 (s). ESI-MS *m*/*z:* 515.14344 [M+H]⁺. HR-ESI-MS spectrum was shown in FIG. 16.

### 30. Compound 31

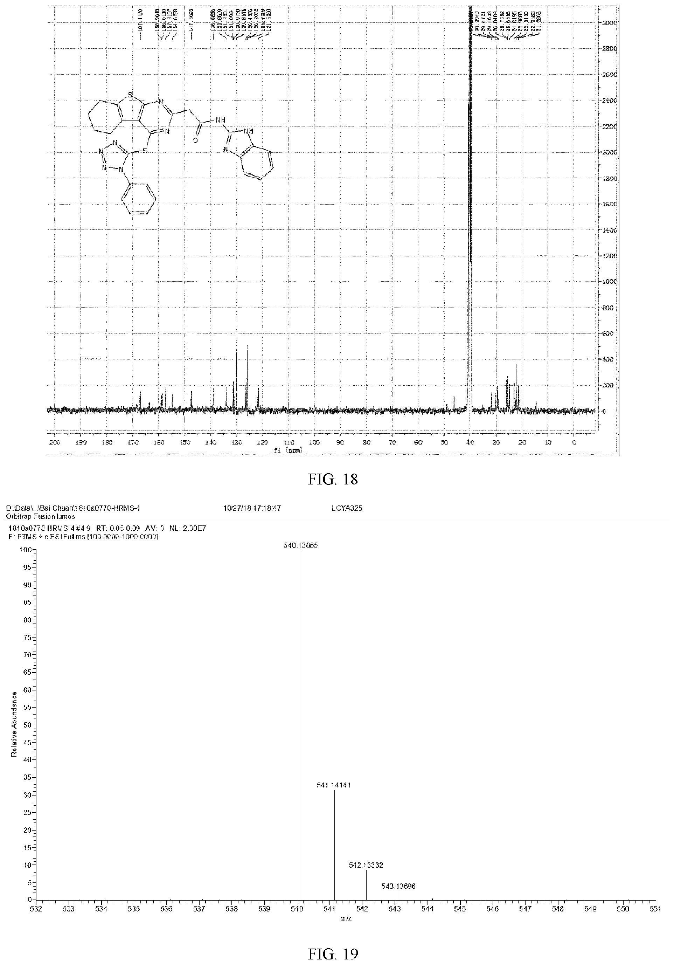

Compound 29 (0.28 mmol), 2-aminobenzimidazole (0.57 mmol), HOBT (0.31 mmol), and EDCI (0.31 mmol) were added into a 100 mL evaporating flask and dissolved in 10 mL of ultra-dry DCM solvent. The reaction solution was stirred at room temperature overnight, then evaporated to dryness by rotary evaporation under reduced pressure. The residue was extracted with ethyl acetate, washed with a saturated salt solution, and the organic layer was evaporated to dryness by rotary evaporation under reduced pressure, then the residue was separated and purified by column chromatography (cyclohexane:acetone = 10:1) to obtain a Compound 31 (yellow solid, 59% yield); Yield=29%. Test result: ¹H NMR spectrum and ¹³C NMR spectrum are shown in FIG. 17 and FIG. 18, respectively. ¹H NMR (500 MHz, DMSO-d₆) δ 7.72 - 7.65 (m, 2H), 7.54 - 7.33 (m, 5H), 7.12 - 7.02 (m, 2H), 3.93 - 3.86 (m, 2H), 3.00 - 2.90 (m, 2H), 2.86 - 2.79 (m, 2H), 1.88 - 1.75 (m, 4H). ¹³C NMR (126 MHz, DMSO-d₆) δ 167.12 (s), 158.96 (s), 158.61 (s), 157.32 (s), 154.68 (s), 147.37 (s), 138.89 (s), 133.86 (s), 131.10 (t, *J* = 15.5 Hz), 129.86 (s), 128.03 - 126.57 (m), 126.44 (s), 125.97 (d, *J=* 59.1 Hz), 121.54 (s), 31.62 (s), 30.29 (s), 29.47 (s), 29.16 (s), 26.05 (s), 25.82 - 24.54 (m), 22.99 (s), 22.29 (d, *J* = 6.9 Hz), 21.29 (s). ESI-MS *m*/*z:* 540.13865 [M+H]⁺. HR-ESI-MS spectrum was shown in FIG. 19.

### II. Test for inhibitory effect

EC₅₀ and CC₅₀ tests: EC₅₀ (half maximal (50%) effective concentration) is a half maximal effective concentration against RORyt-LBD+-Jurkat cells, and CC₅₀ (median (50%) cytotoxic concentration) is a median cytotoxic concentration against RORyt-LBD+-Jurkat cells. The results are shown in Tables 1, 2 and 3 below.

**Table 1**

| Compound | R₁ | R₂ | EC₅₀ (*µ*M) | CC₅₀ (*µ*M) | clogP |
|---|---|---|---|---|---|
| 6 | H | H | 2.79 | 3.98 | 4.01 |
| 7 | Me | H | >10 | >10 | 4.25 |
| 8 | Et | H | >10 | >10 | 4.46 |
| 9 | Pro | H | >10 | >10 | 5.03 |
| 10 | Me | Me | 4.58 | 7.70 | 4.64 |
| 11 | 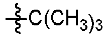 | H | 8.25 | <1 | 4.64 |
| 12 | F | F | 1.35 | <1 | 3.96 |
| 13 | 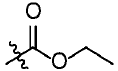 | H | 6.15 | >10 | 3.80 |
| 14 | 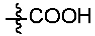 | H | >20 | >10 | 3.32 |

| | | | | | |
|---|---|---|---|---|---|
| Note: clogP was calculated by LigandScout 4.2, the same below. | | | | | |

**Table 2**

| Compound | R₄ | EC₅₀ (*µ*M) | CC₅₀ (*µ*M) | clogP |
|---|---|---|---|---|
| 15 | 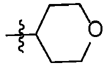 | >10 | <10 | 3.53 |
| 16 | 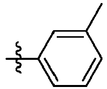 | >10 | <10 | 5.18 |
| 17 | 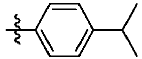 | >10 | <1 | 5.99 |
| 18 | 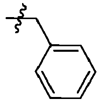 | 12.60 | >10 | 4.26 |
| 19 | 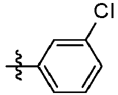 | >10 | <10 | 5.52 |
| 20 | 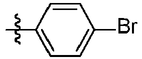 | >10 | <10 | 5.63 |
| 21 | 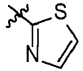 | >10 | <10 | 4.33 |
| 22 | 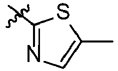 | 9.06 | 4.32 | 4.64 |
| 23 | 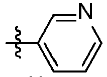 | >10 | <5 | 4.27 |
| 24 | 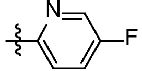 | >10 | <10 | 4.40 |
| 25 | 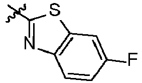 | >10 | <5 | 5.62 |

**Table 3**

| Compound | R₃ | EC₅₀ (*µ*M) | CC₅₀ (*µ*M) | clogP |
|---|---|---|---|---|
| 28 | 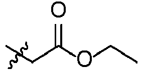 | 0.14 | 3.35 | 3.80 |
| 29 | 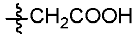 | >10 | >10 | 3.32 |
| 30 | 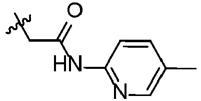 | >10 | 4.014 | 4.58 |
| 31 | 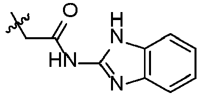 | 0.85 | 9.176 | 4.76 |

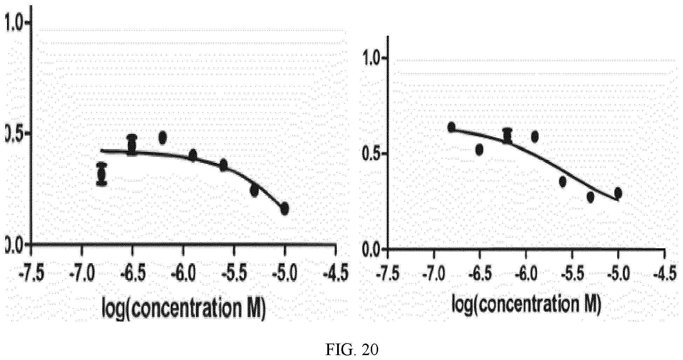

FIG. 20 shows the results of RORyt transcription activity inhibited by compounds 28 and 31. Compound 28 (left): EC50 = 0.14 µM. Compound 31 (right): EC50 = 0.85 µM. It can be seen from FIG. 20 that Compounds 28 and 31 have a significant effect on inhibiting RORyt transcription activity.

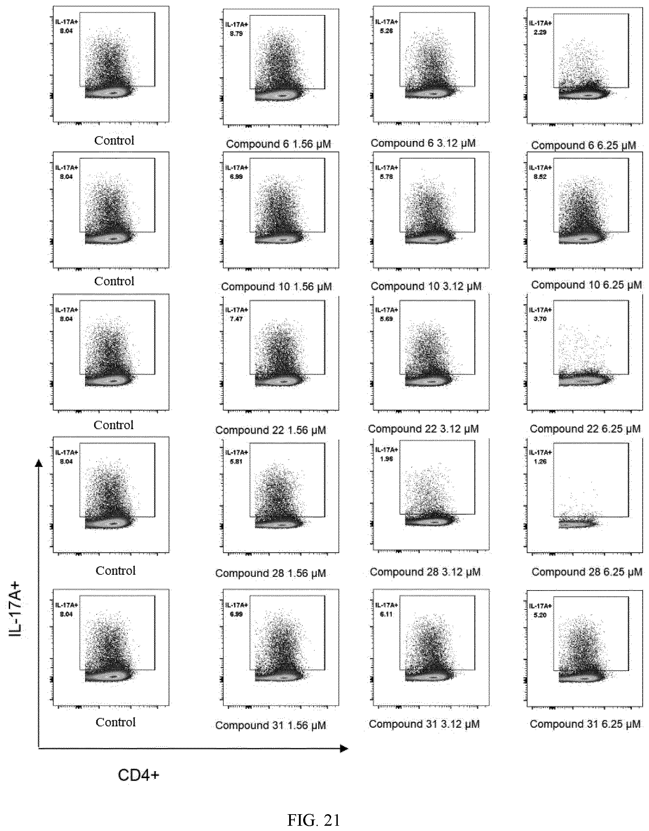

FIG. 21 shows data of intracellular protein expression of IL-17A inhibited by Compounds 6, 10, 22, 28, and 31. It can be seen from FIG. 21 that Compounds 6, 10, 22, 28, and 31 have significant inhibitory effects. In the figure, the results of solvent group, 1.56 µM compound group, 312 µM compound group, and 6.25 µM compound group are shown from left to right.

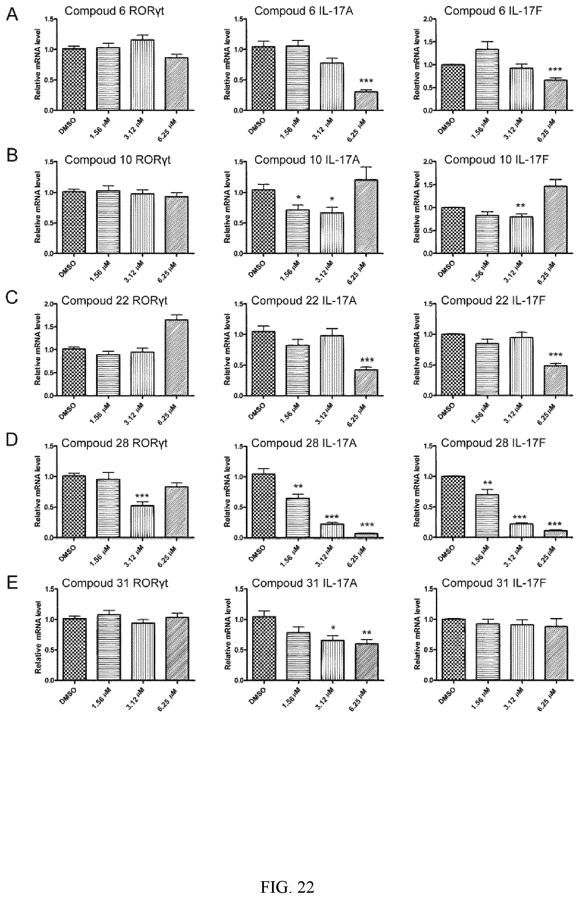

FIG. 22 shows results of mRNA expression of RORyt (left), IL17A (middle), and IL-17F (right) inhibited by Compounds 6, 10, 22, 28, and 31 in the differentiation induced by Th17 cells in vitro. In the figure, the results of solvent group, 1.56 µM compound group, 312 µM compound group, and 6.25 µM compound group are shown from left to right. It can be seen from FIG. 22 that Compounds 6, 10, 22, 28, and 31 have significant inhibitory effects.

In addition, similar experiments were performed on other compounds. Although the results were slightly worse than those of Compounds 6, 10, 22, 28, and 31, significant effects on inhibiting RORyt transcription activity can also be observed as the dosage and concentration increased.

Each of the technical features of the above-mentioned embodiments may be combined arbitrarily. To simplify the description, not all the possible combinations of each of the technical features in the above embodiments are described. However, all of the combinations of these technical features should be considered as within the scope of this disclosure, as long as such combinations do not contradict with each other.

The above-mentioned embodiments are merely illustrative of several embodiments of the present disclosure, which are described specifically and in detail, but it cannot be understood to limit the scope of the present disclosure. It should be noted that, for those ordinary skilled in the art, several variations and improvements may be made without departing from the concept of the present disclosure, and all of which are within the protection scope of the present disclosure. Therefore, the protection scope of the present disclosure shall be defined by the appended claims.

## Claims

1. A compound for inhibiting RORyt activity having the following structural formula (A): 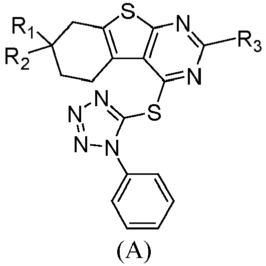 where R₁ and R₂ are one each independently selected from the group consisting of hydrogen, fluorine, chlorine, bromine, iodine, a C1 to C4 linear or branched alkyl group, 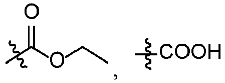 and 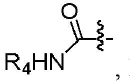 R₄ is selected from the group consisting of 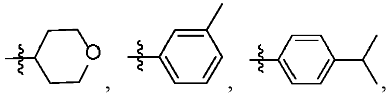 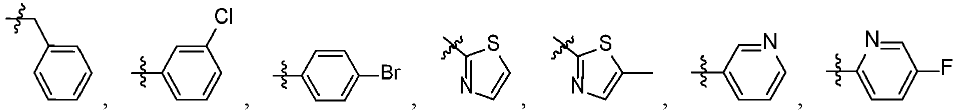 and 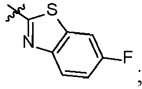 and R₃ is selected from the group consisting of methyl,

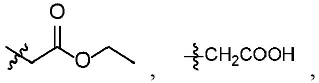

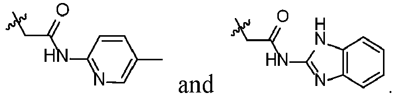

2. The compound for inhibiting RORyt activity according to claim 1, wherein R₃ is methyl, R₁ and R₂ are not simultaneously hydrogen, and when one of R₁ and R₂ is hydrogen, the other is not methyl.

3. The compound for inhibiting RORyt activity according to claim 2, wherein one of R₁ and R₂ is hydrogen, and the other is selected from the group consisting of a C2 to C4 linear or branched alkyl group, 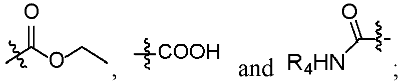 or R₁ and R₂ are both methyl; or
R₁ and R₂ are independently selected from the group consisting of fluorine, chlorine, bromine and iodine.

4. The compound for inhibiting RORyt activity according to claim 3, wherein R₁ and R₂ are simultaneously hydrogen, and R₃ is selected from the group consisting of

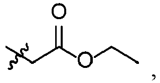

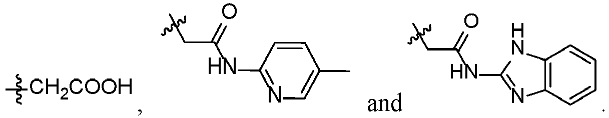

5. The compound for inhibiting RORyt activity according to claim 1, wherein the compound represented by the structural formula (A) is one selected from the following compounds:

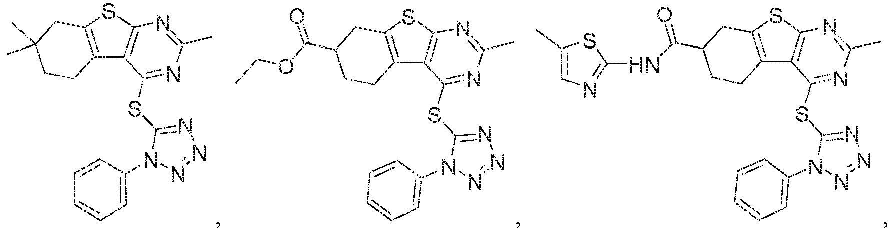

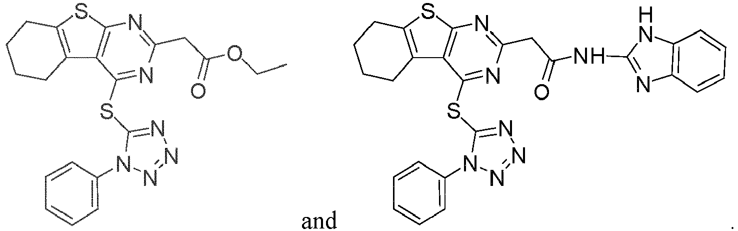

6. A method for preparing a compound for inhibiting RORyt activity, comprising the following steps: reacting compounds 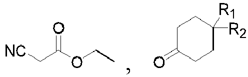 with elemental sulfur in the presence of morpholine and ethanol to prepare a compound 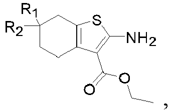 where R₁ and R₂ are each independently selected from the group consisting of hydrogen, fluorine, chlorine, bromine, iodine, a C1 to C4 linear or branched alkyl group, 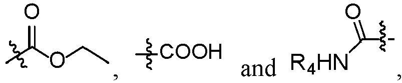 R₄ is selected from the group consisting of 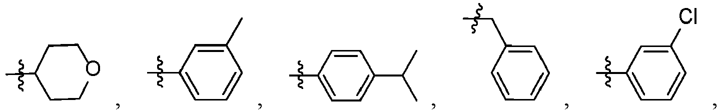 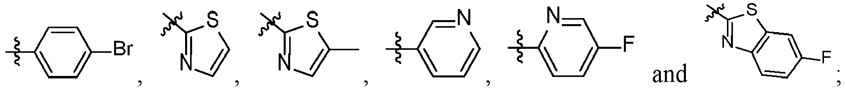 reacting the prepared compound 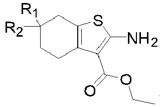 with NC-R₃ in a solution of hydrogen chloride in 1,4-dioxane to prepare a compound 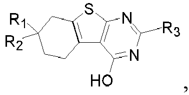 wherein R₃ is selected from the group consisting of methyl, 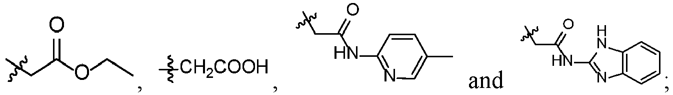 reacting the prepared compound 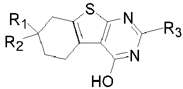 with phosphorus oxychloride to prepare a compound 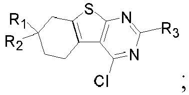 and reacting the prepared compound 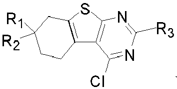 with a compound 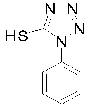 in the presence of sodium bicarbonate and dimethyl sulfoxide to prepare a compound

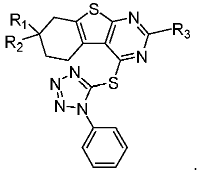

7. The method for preparing a compound for inhibiting RORyt activity according to claim 6, wherein the compound 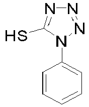 is prepared by reacting phenyl thioisocyanate and sodium azide in an aqueous solution.

8. The method for preparing a compound for inhibiting RORyt activity according to claim 6, wherein one of R₁ and R₂ is hydrogen, and the other is 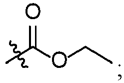 and the method further comprises reacting a compound 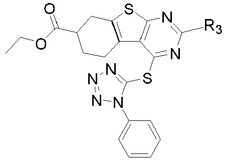 in a lithium hydroxide aqueous solution to prepare a compound

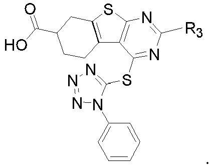

9. The method for preparing a compound for inhibiting RORyt activity according to claim 8, wherein, one of R₁ and R₂ is hydrogen, and the other is 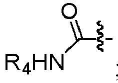 ; and the method further comprises reacting the prepared compound 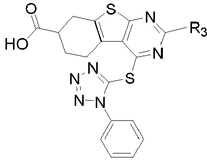 with R₄NH₂, HOBT and EDCI in an ultra-dry DCM solvent to prepare a compound

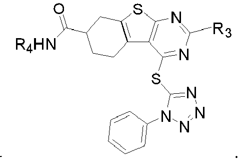

10. Use of the compound for inhibiting RORyt activity according to any one of claims 1 to 5 in preparation of a therapeutic formulation for treatment of autoimmune diseases, in preparation of a formulation that inhibits formation of a Th17 cell, in preparation of a formulation that inhibits RORyt transcription activity, or in preparation of a formulation that inhibits transcription and expression of IL17A gene and/or IL17F gene.
